# EUROPEAN PATENT APPLICATION

(11) **EP 3 682 805 A1**
(43) Date of publication of application: **22.07.2020**
(21) Application number: 20152700.9
(22) Date of filing: 20.01.2020
(51) Int. Cl.: A61B 6/00

(54) **PORTABLE EXPANSION MODULE, X-RAY DETECTOR, AND CONTROL METHOD OF X-RAY DETECTOR**

(30) Priority: 18.01.2019 KR 20190007138
(71) Applicant: Samsung Electronics Co., Ltd., Gyeonggi-do 16677 (KR)
(72) Inventor: KIM, Jeong Sik, 16677 Gyeonggi-do (KR); KIM, Sung Nam, 16677 Gyeonggi-do (KR); LEE, Dong Hun, 16677 Gyeonggi-do (KR)
(74) Representative: D'Halleweyn, Nele Veerle Trees Gertrudis

(57) **Abstract**

Disclosed herein is a portable expansion module, an X-ray detector, and a method of controlling the X-ray detector. The portable expansion module includes a connecting portion configured to connect to a connector of an X-ray detector, and a display configured to operate using power supplied from the X-ray detector to the portable expansion module based on the connecting portion being connected to the connector, receive data from the X-ray detector based on the connecting portion being connected to the connector, and display state information of the X-ray detector based on the data received from the X-ray detector.

## Description

The disclosure relates to a portable expansion module configured to provide an expanded function to an X-ray detector, an X-ray detector, and a method of controlling the X-ray detector.

In medical practice, a clinical diagnosis provides a major role in the treatment of patients. Advances in medical technology are improving the accuracy of clinical diagnosis, and are increasing the amount of dependence on clinical diagnoses.

Accordingly, image diagnosis apparatuses such as computer tomography (CT) scanners, magnetic resonance imaging (MRI) scanners, and X-ray imaging apparatuses have become ubiquitous in modern medical practice.

An X-ray detector of an X-ray imaging apparatus is a device that images an internal structure of an object by detecting X-rays that pass through the object. Based on the X-ray detector detecting X-rays and converting the X-rays into electrical signals, a host device of the X-ray imaging apparatus may process the electrical signals to generate an X-ray image representing the anatomy of the object.

Recently, wireless X-ray detectors have been developed and used. Because a wireless X-ray detector may be attached to and detached from the X-ray imaging apparatus, the X-ray image may be wirelessly transmitted to a workstation. However, conventional X-ray detectors may lack the ability to be modularized because conventional wireless X-ray detectors may have been designed based on the "Bucky system," and the workstation may be configured to operate only in an internal access point (AP) mode.

Therefore, it is an aspect of the disclosure to provide an expansion module configured to provide additional functionality to a wireless X-ray detector, and further provide an X-ray detector configured to increase mobility and usability because the wireless X-ray detector may directly communicate with a user terminal, and a control method of the X-ray detector.

Additional aspects of the disclosure will be set forth in part in the description which follows and, in part, will be obvious from the description, or may be learned by practice of the disclosure.

According to an aspect of the disclosure, a portable expansion module may include a connecting portion configured to connect to a connector of an X-ray detector, and a display configured to operate using power supplied from the X-ray detector based on the connecting portion being connected to the connector, receive data from the X-ray detector based on the connecting portion being connected to the connector, and display state information of the X-ray detector based on the data received from the X-ray detector.

The portable expansion module may include an input component configured to receive an input command from a user, and the input component may transmit the input command identifying a change in a software enabled access point (Soft AP) mode to the X-ray detector.

The portable expansion module may include an input component configured to receive an input command from a user, and the input component may transmit the input command identifying an Automatic Exposure Detection (AED) function to the X-ray detector.

The display may display state information including at least one of a switching of a Soft AP mode of the X-ray detector, an AED standby state, a number of possible AED images, a patient identifier (ID), and an imaging number, based on the data received from the X-ray detector.

The portable expansion module may include a memory configured to store the data received from the X-ray detector.

The portable expansion module may include a communication circuitry configured to receive a search request signal from a workstation; and transmit a search response signal in response to the search request signal to the workstation.

According to an aspect of the disclosure, an X-ray detector may include a battery; a connector configured to connect to at least one of a cable or a portable expansion module; a detector configured to detect X-rays that are irradiated from an X-ray source; and a controller configured to detect that the X-ray detector is connected to the portable expansion module; supply power to the portable expansion module from the battery based on detecting that the X-ray detector is connected to the portable expansion module; and transmit data associated with state information of the X-ray detector to the portable expansion module to permit the portable expansion module to display the state information.

The controller may switch from an access point (AP) mode to a software enabled access point (Soft AP) mode based on detecting that the X-ray detector is connected to the portable expansion module.

The controller may switch from the AP mode to the Soft AP mode based on an input command transmitted via the portable expansion module.

The controller may control the detector to perform an Automatic Exposure Detection (AED) function based on an input command transmitted via the portable expansion module; and display state information regarding an AED standby state via the portable expansion module.

The X-ray detector may include a communication circuitry configured to communicate with a user terminal that is external to the X-ray detector, and controller may identify the user terminal that is connected to the X-ray detector via the communication circuitry, based on the Soft AP mode.

The controller may transmit the X-ray image to the user terminal via the communication circuitry.

The X-ray detector may include a storage configured to store the X-ray image, and the controller may assign an imaging number to the X-ray image stored in the storage.

The controller may display state information including at least one of a number of possible AED images, the imaging number, and a patient identifier (ID) associated with the stored X-ray image, via the portable expansion module.

A method of controlling an X-ray detector may include detecting that a portable expansion module is connected to the X-ray detector via a connector; supplying power to the portable expansion module from a battery based on detecting that the portable expansion module is connected to the X-ray detector; transmitting data associated with state information of the X-ray detector to the portable expansion module; and controlling the portable expansion module to display the state information.

The method may include switching from an access point (AP) mode to a software enabled access point (Soft AP) mode based on detecting that the portable expansion module is connected to the X-ray detector.

The method may include switching from an AP mode into a Soft AP mode based on an input command transmitted via the portable expansion module.

The method may include controlling a detector to perform an Automatic Exposure Detection (AED) function based on the input command transmitted via the portable expansion module, and the displaying may include displaying state information associated with an AED standby state, via the portable expansion module.

The method may include converting X-rays detected by the detector into an X-ray image, based on performing the AED function, and displaying state information associated with a completion of the X-ray imaging, via the portable expansion module.

The method may include identifying a user terminal connected to the X-ray detector via communication circuitry, based on a Soft AP mode; and transmitting the X-ray image to the user terminal via the communication circuitry.

The method may include storing the X-ray image, assigning an imaging number to the X-ray image stored in the storage, and displaying state information including at least one of a number of possible AED images, the imaging number, and a patient identifier (ID) associated with the stored X-ray image, via the portable expansion module.

The above and other aspects, features, and advantages of certain embodiments of the present disclosure will be more apparent from the following description taken in conjunction with the accompanying drawings, in which:
FIG. 1 is a view of an appearance of a portable expansion module, an X-ray detector, and a user terminal according to an ;
FIG. 2A is a control block diagram of a portable expansion module, an X-ray detector, and a user terminal according to an embodiment;
FIG. 2B is a view illustrating a detector of the x-ray detector according to an embodiment;
FIG. 3 is a view illustrating a software enabled access point (Soft AP) mode according to an embodiment;
FIG. 4 is a view illustrating the Soft AP mode according to an embodiment;
FIG. 5 is a flow chart illustrating an operation of the portable expansion module according to an embodiment;
FIG. 6 is a view illustrating the portable expansion module operating as a storage medium according to an embodiment;
FIG. 7 is a flow chart illustrating a method for changing a communication mode of the X-ray detector according to an embodiment;
FIG. 8 is a view illustrating an example of FIG. 7 according to an embodiment;
FIG. 9 is a view illustrating the example of FIG. 7 according to an embodiment;
FIG. 10 is a flowchart illustrating an X-ray imaging method of the X-ray detector according to an embodiment;
FIG. 11 is a view illustrating an example of FIG. 10 according to an embodiment;
FIG. 12 is a view illustrating the example of FIG. 10 according to an embodiment;
FIG. 13 is a control block diagram of a portable expansion module according to an embodiment;
FIG. 14 is an external view illustrating an X-ray imaging apparatus including the portable expansion module according to an embodiment; and
FIG. 15 is a flow chart illustrating a function provided by the portable expansion module according to an embodiment.

In the following description, like reference numerals may refer to like elements throughout the specification. Well-known functions or constructions might not be described in detail so as to not obscure the disclosure with unnecessary detail. Terms such as "unit," "module," "member," "block," etc., may refer to elements that may be embodied as hardware, software, and/or a combination of hardware and software. According to an embodiment, a plurality of "units," "modules," "members," "blocks," etc., may be implemented as a single component or as a plurality of components.

It should be understood that when an element is described as being "connected to" another element, the element may be directly or indirectly connected to the other element, and the indirect connection may include a connection via a wireless communication network.

Also, when a component is described as "including" or "comprising" an element, the component may include other elements unless the context explicitly indicates otherwise.

Throughout the description, when a member is described as being "on" another member, the member may directly contact the other member, or another member may be disposed between the two members such that the two members are indirectly in contact.

It should be understood that, although the terms such as "first," "second," "third," etc., may describe various elements, but the elements should not be limited by these terms. These terms may be used to distinguish an element from another element.

As used herein, the singular forms of terms such as "a," "an," and "the," may include the plural forms of the terms as well, unless the context clearly indicates otherwise.

An identification code may be used for convenience of description, but is not intended to illustrate the order of each step. The steps may be implemented in a different order from the illustrated order unless the context clearly indicates otherwise.

Hereinafter, embodiments of a portable expansion module, an X-ray detector, a method of controlling an X-ray detector, a workstation, and an X-ray imaging apparatus according to an aspect of the disclosure will be described in detail with reference to the accompanying drawings.

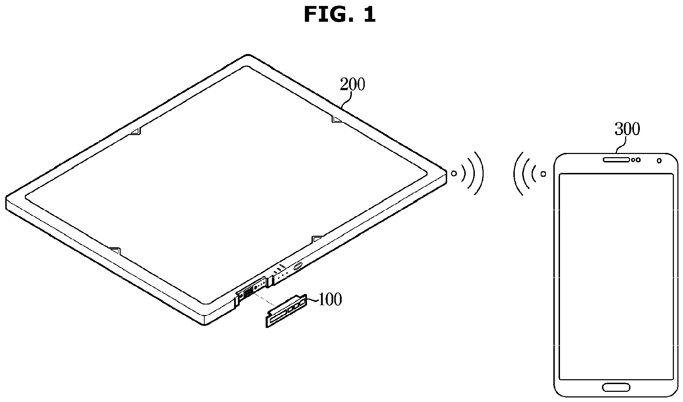

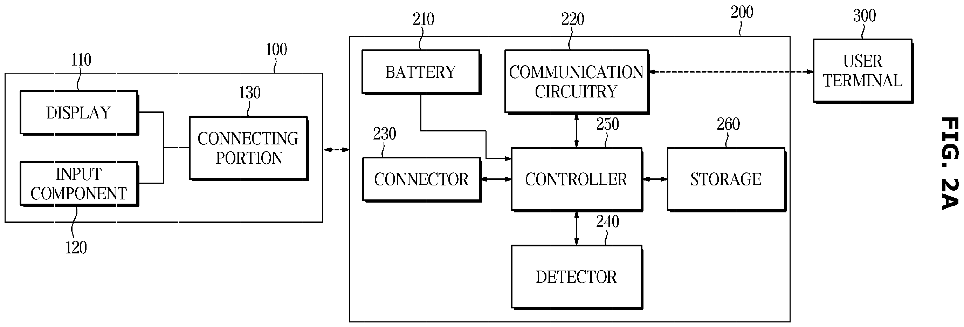

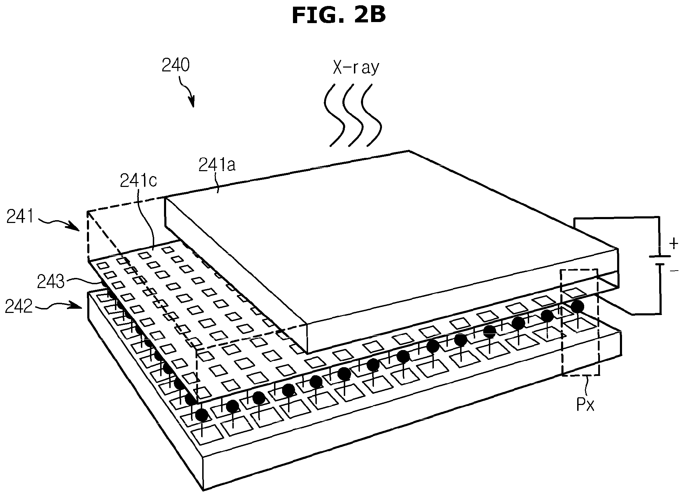

FIG. 1 is a view of an appearance of a portable expansion module, an X-ray detector, and a user terminal according to an embodiment of the disclosure, FIG. 2A is a control block diagram illustrating a configuration of FIG. 1, and FIG. 2B is a view illustrating a configuration of a detector of the x-ray detector. In order to reduce redundant description, FIGS. 1, 2A, and 2B will be described together.

Referring to FIG. 1, an X-ray detector 200 may be connected to a portable expansion module 100, and the X-ray detector 200 that is connected to the portable expansion module 100 may communicate with a device that is external to the X-ray detector 200 such as a user terminal 300.

Referring to FIG. 2B, in order to provide or enhance a variety of functions of the X-ray detector 200, the portable expansion module 100 may include a display 110 configured to display state information transmitted from the X-ray detector 200, an input component 120 configured to receive an input command from a user, and a connecting portion 130 configured to connect to the X-ray detector 200.

Particularly, the display 110 of the portable expansion module 100 may display state information based on data transmitted from the X-ray detector 200. The state information may include various information such as whether an access point (AP) mode of the X-ray detector 200 is changed, an Automatic Exposure Detection (AED) standby state, the number of possible AED images, a patient identifier (ID), an imaging number, or the like.

The display 110 of the portable expansion module 100 may be provided as a light-emitting diode (LED) panel, or may be provided as various display panels that are mobile.

The input component 120 may receive various input commands related to the operation of the X-ray detector 200 from a user. The user may be a person who acquires an X-ray image of the object using the X-ray detector 200, and may be medical personnel such as a doctor, a radiologist, a nurse, or the like. Therefore, anyone who can use the X-ray detector 200 and the X-ray imaging apparatus 1 may be a "user," as described herein.

An input command may include a command associated with X-ray imaging, such as an AED function, a command associated with communication such as a software enabled access point (Soft AP) mode, or the like. Various input commands received by the portable expansion module 100 and the operation of the X-ray detector 200 based on the input commands will be described in more detail elsewhere herein.

The input component 120 may be provided as a button configured to receive a user's input command. In addition, the input component 120 may be implemented as a mobile hardware device such as a track ball, a lever, a handle, a joy stick, a switch, or the like. The display 110 of the portable expansion module 100 may be implemented as a touch screen panel (TSP), and the display 110 may operate as the input component 120. Accordingly, the input component 120 may be omitted in some cases.

The connecting portion 130 may be configured to connect to a connector 230 of the X-ray detector 200. After being connected to the connector 230, the connecting portion 130 may transmit a connection signal to the X-ray detector 200. In addition, the connecting portion 130 may receive power supplied via the connector 230 of the X-ray detector 200 based on the connection signal. The power received via the connecting portion 130 may be used for the operation of the portable expansion module 100, such as the display 110 and the input component 120.

The connecting portion 130 may receive power, may receive data from the X-ray detector 200, and may transmit the input information received by the input component 120 to the X-ray detector 200.

The connecting portion 130 may be connected to the connector 230 that is used when the X-ray detector 200 is connected to a cable provided in a body of the X-ray imaging apparatus 1. For example, when the connector 230 of the X-ray detector 200 is provided as a male connector including a plurality of pins, the connecting portion 130 may be provided in the form of a female connector that accommodates the male connector. As another example, when the connector 230 is provided as a female connector, the connecting portion 130 may be provided in the form of a male connector.

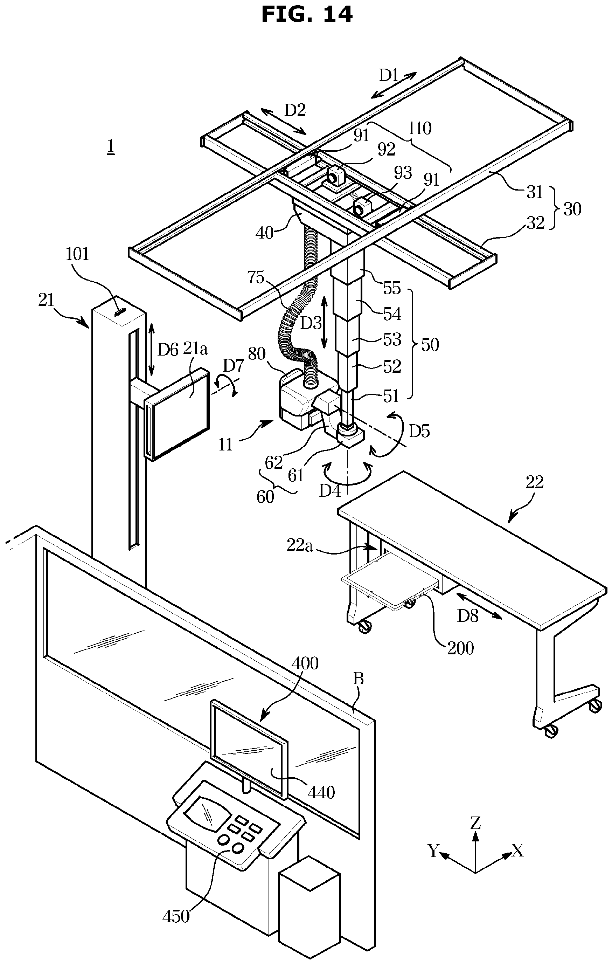

The portable expansion module 100 may further include various components in addition to the above-described components based on desired functionality of the portable expansion module 100, which will be described later with reference to FIG. 14.

The X-ray detector 200 includes a battery 210, a communication circuitry 220, the connector 230, a detector 240, a controller 250, and a storage 260.

The battery 210 may supply power to the X-ray detector 200 as described above. The X-ray detector 200 may be separated from the X-ray imaging apparatus 1, may be carried by a user, and may exchange data with the X-ray imaging apparatus 1 via wireless communication. In this case, the X-ray detector 200 may include the battery 210 that may operate via its own power source, and the power of the battery 210 may be supplied to the connected portable expansion module 100.

The communication circuitry 220 may be configured to communicate with a device that is external to the X-ray detector 200. For example, the communication circuitry 220 may communicate with the user terminal 300, the X-ray imaging apparatus 1, the workstation 400, or the like.

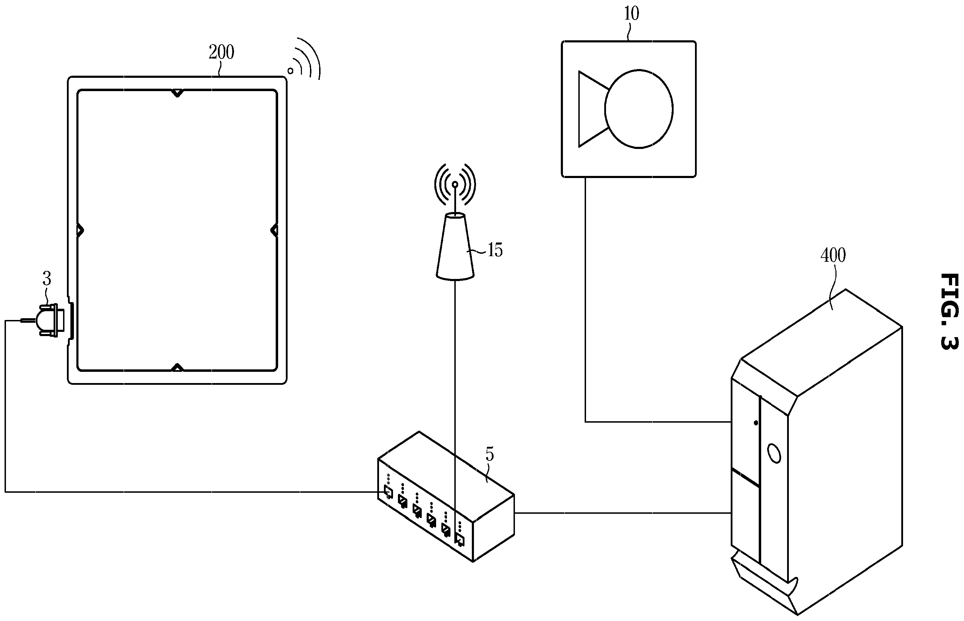

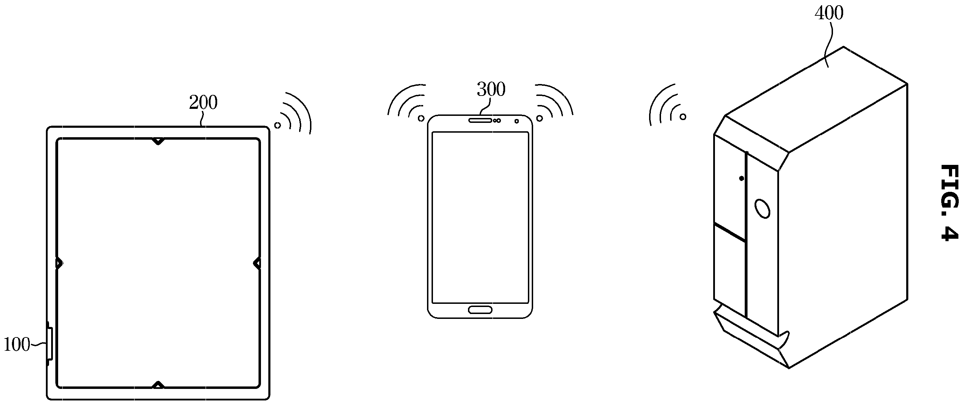

The X-ray detector 200 may perform wireless communication with the X-ray imaging apparatus 1 and the workstation 400 via an AP mode. In the AP mode, data may be exchanged via an internal wireless access point (AP) provided in the X-ray imaging apparatus 1 or the workstation 400. However, when the portable expansion module 100 is connected to the X-ray detector 200 according to an embodiment, the communication circuitry 220 may be switched to the Soft AP mode. A detailed description of the Soft AP mode will be described in more detail elsewhere herein with reference to FIGS. 3 and 4.

The communication circuitry 220 may include at least one of a short-range communication module, a wired communication module, and a wireless communication module.

The short-range communication module may include various short-range communication modules configured to transmit and receive signals using short-range wireless communication, and may be a Bluetooth module, an infrared communication module, a radiofrequency identification (RFID) communication module, a wireless local access network (WLAN) communication module, a near field communication (NFC) communication module, a ZigBee communication module, or the like. When the X-ray detector 200 operates in the Soft AP mode, the communication circuitry 220 may implement the Soft AP mode using the short-range communication module.

The wired communication module may include a local area network (LAN) module, a wide area network (WAN) module, a value added network (VAN) module, a universal serial bus (USB) module, a High Definition Multimedia Interface (HDMI) module, a Digital Visual Interface (DVI) module, a recommended standard 232 (RS-232) module, a power line communication module, a plain old telephone service (POTS) module, or the like.

The wireless communication module may include a Wi-Fi module and the wireless broadband module, and may further include a module configured to support a variety of wireless communication methods such as global system for mobile communication (GSM), code division multiple access (CDMA), wideband code division multiple access (WCDMA), universal mobile telecommunications system (UMTS), time division multiple access (TDMA), long term evolution (LTE), fifth generation new radio (5G NR), or the like.

The wireless communication module may include a wireless communication interface including an antenna and a transmitter configured to transmit a signal generated by the X-ray detector 200. In addition, the wireless communication module may further include a signal conversion module configured to modulate a digital control signal, which is output from the controller 250 via the wireless communication interface under the control of the controller 250, into an analog type wireless signal.

The wireless communication module may include a wireless communication interface including an antenna and a receiver configured to receive a signal transmitted from the X-ray imaging apparatus 1 or the workstation 400. In addition, the wireless communication module may further include a signal conversion module configured to demodulate an analog type wireless signal, which is received via a wireless communication interface, into a digital control signal.

The detector 240 may be configured to receive X-rays.

Referring to FIG. 2B, the detector 240 may include a light receiving element 241 configured to generate an electrical signal by detecting X-rays, and a readout circuit 242 configured to read out the generated electrical signal. According to an embodiment, the controller 250 may process the electrical signal output from the readout circuit 242, and may generate X-ray image data constituting the X-ray image.

The generated X-ray image data may be stored in the storage 260 together with (or separately from) detector state information or X-ray imaging information as described in more detail elsewhere herein.

A single crystal semiconductor material may be used as the light receiving element 241 to permit high resolution, fast response time, and high dynamic range at low energy and low dose, and the single crystal semiconductor material may include Germanium (Ge), Cadmium telluride (CdTe), Cadmium zine telluride (CdZnTe), Gallium arsenide (GaAs), or the like.

The light receiving element 241 may form a PIN photodiode in which a p-type semiconductor substrate 241c having a two-dimensional array structure is bonded to a lower portion of an n-type semiconductor substrate 241a having a high resistance.

The readout circuit 242 using a complementary metal oxide semiconductor (CMOS) process forms a two-dimensional array structure and may be connected to the p-type substrate 241c of the light receiving element 241 for each pixel Px. In this case, a bonding method may be a flip chip bonding method in which bumps 243 such as solder (PbSn) or indium (In) is formed and then reflowed, heated, and pressed.

The X-ray detector 200 may perform an automatic exposure detection (AED) function. The AED function may refer to monitoring for the presence or absence of X-rays that may be received in a predetermined region among all regions of the X-ray detector 200 in which the light receiving elements 241 are arranged.

Particularly, the light receiving element 241 may convert the X-rays into light based on the received X-rays being greater than or equal to a predetermined size. The readout circuit 242 may generate an electrical signal using the light that is converted by the light receiving element. However, because the X-ray detector 200 may be an independent structure that is not controlled by the X-ray imaging apparatus 1, it may be difficult to accurately estimate a point in time at which the X-ray imaging apparatus 1 will irradiate X-rays. Therefore, the X-ray detector 200 may be provided with a sensor that allocates a predetermined pixel or several lines among all the pixels Px of the light receiving element 241 to detect the X-rays before receiving the X-rays. That is, via a sensor, the X-ray detector 200 may monitor for and detect a point in time at which X-rays are irradiated, and the X-ray detector 200 may operates the entirety of the light receiving element 241 and the readout circuit 242 based on detecting that the X-rays are irradiated.

The AED function may be divided into an AED standby in which a preparation for receiving X-rays is completed after monitoring the irradiated X-rays, and an AED process, which may be a series of processes for generating an electrical signal after converting the received X-rays to light. State information related to the AED function may be displayed via the portable expansion module 100.

The sensor configured to perform the AED function may include several lines as illustrated in FIG. 2B, but is not limited thereto. Therefore, a sensor may be implemented in a different manner than as shown in FIG. 2B.

Referring to FIG. 2A, the controller 250 may be implemented as a memory configured to store data associated with controlling the operation of the components in the X-ray detector 200, and a processor configured to perform the operations described herein using the data stored in the memory. In this case, the memory and the processor may be implemented as separate chips. Alternatively, the memory and the processor may be implemented as a single chip. The processor may include a processor (e.g., a central processing unit (CPU), a graphics processing unit (GPU), an accelerated processing unit (APU), etc.), a microprocessor, and/or any processing component (e.g., a field-programmable gate array (FPGA), an application-specific integrated circuit (ASIC), etc.) that interprets and/or executes instructions. The memory may include a random access memory (RAM), a read only memory (ROM), and/or another type of dynamic or static storage device (e.g., a flash memory, a magnetic memory, an optical memory, etc.) that stores information and/or instructions for use by the processor.

The controller 250 may identify whether the portable expansion module 100 is connected to the connector 230, and may control the communication circuitry 220 to operate in the Soft AP mode based on the portable expansion module 100 being connected to the connector 230. Further, the controller 250 may control the communication circuitry 220 in the Soft AP mode based on a user's input command transmitted from the portable expansion module 100.

The controller 250 may perform an AED function and signal processing on an electrical signal transmitted from the detector 240, and thereby may generate an X-ray image. The controller 250 may store the X-ray image in the storage 260. In addition, the controller 250 may display state information on the stored X-ray image via the portable expansion module 100, and may transmit the stored X-ray image to the user terminal 300 and/or the workstation 400 via the communication circuitry 220.

A detailed description of the control method of the controller 250 will be described later in more detail elsewhere herein.

As described above, the storage 260 may store various programs and data for generating the X-ray image, and for the operation of the X-ray detector 200.

The storage 260 may be implemented by at least one of a non-volatile memory device such as a ROM, a programmable ROM (PROM), an erasable programmable ROM (EPROM), an electrically erasable programmable ROM (EEPROM), and a flash memory, a volatile memory device such as a RAM, or a storage medium such as a hard disk drive (HDD) and a CD-ROM, but is not limited thereto. The storage 260 may be a memory implemented as a chip separate from the processor described above with respect to the controller 250, or may be implemented as a single chip with the processor.

The user terminal 300 may directly receive an X-ray image from the X-ray detector 200.

Particularly, the user terminal may be a computer or a portable terminal configured to connect to the X-ray detector 200 via a wireless network. For example, the user terminal may include a laptop, a desktop, a laptop, a tablet personal computer (PC), a slate PC, or the like, which is equipped with a web browser. The portable terminal may be a wireless communication device that provides portability and mobility, and may include a handheld-based wireless communication device such as a personal communication system (PCS), a global system for mobile communications (GSM), a personal digital cellular (PDC), a personal handy-phone system (PHS), a personal digital assistant (PDA), an international mobile telecommunication (IMT)-2000 device, a code division multiple access (CDMA) -2000 device, a w-code division multiple access (WCDMA) device, a wireless broadband Internet (WiBro) terminal, a smart phone, and a wearable device such as a watch, a ring, a bracelet, an anklet, a necklace, glasses, contact lenses, a head-mounted-device (HMD), or the like.

That is, the user terminal 300 according an embodiment may be a device that displays an X-ray image transmitted by the X-ray detector 200 to a user, such as a smart phone or a tablet.

The user terminal 300 may include a large-capacity storage medium configured to receive the X-ray image transmitted by the X-ray detector 200 wirelessly, and may store the X-ray image received from the X-ray detector 200 via the Soft AP.

FIGS. 3 and 4 are views illustrating a software enabled access point (Soft AP) mode. In order to reduce redundant description, FIGS. 3 and 4 will be described together.

Referring to FIG. 3, in a conventional Bucky system, the X-ray detector 200 may be wirelessly connected to an AP router 15, and may connect to the workstation 400 of the X-ray imaging apparatus 1 via the AP router 15.

Particularly, based on the X-ray source 10 controlled by the workstation 400 emitting X-rays, the X-ray detector 200 may receive the X-rays. The X-ray detector 200 may generate an X-ray image using a signal processing technique based on the received X-rays.

The X-ray detector 200 may be connected to a LAN HUB 5 via the main cable 3. The LAN HUB 5 may be connected to the AP router 15. That is, the X-ray detector 200 in the conventional AP mode may wirelessly transmit the X-ray image via the AP router 15 to the workstation 400.

In this AP mode, the X-ray detector 200 may provide portability and mobility. However, when the X-ray detector 200 is not connected to the AP router 15, the X-ray detector 200 might not be capable of transmitting the generated X-ray image to the workstation 400.

Referring to FIG. 4, the X-ray detector 200 may directly transmit an X-ray image to the user terminal 300 via the Soft AP mode. Soft AP may refer to an abbreviation for "software enabled access point," which may refer to a software program that changes a device that does not allow access to a wireless AP into a device that allows access to the wireless AP. In the context of smartphones, Soft AP may be referred to as "tethering" (or "mobile hotspot").

Accordingly, the X-ray detector 200 may switch to the soft AP mode, and may use the user terminal 300 as the AP router 15, and transmit the X-ray image to the workstation 400 via at least one of a 3G network, a Wi-Fi network, an LTE network, a 5G NR network, or the like, provided by the user terminal 300.

According to an embodiment, because the X-ray detector 200 that switches to the Soft AP mode may directly communicate with another X-ray imaging apparatus 1 configured to irradiate X-rays, mobility may be further expanded. For example, after carrying the X-ray detector 200 and the user terminal 300, the user may acquire an X-ray image at substantially any location where a device configured to irradiate X-rays is placed. Based on the x-ray detector 200 generating an X-ray image, the user terminal 300 may directly receive the generated X-ray image, and display the generated X-ray image to the user. That is, the X-ray detector 200 may transmit the X-ray image even in a location where the AP router 15 is not provided, and the user may immediately check the generated X-ray image via the user terminal 300.

The operation of the X-ray detector 200 in the Soft AP mode may be performed based on connecting the portable expansion module 100 or based on a user input. Hereinafter, operations of the portable expansion module 100 and the X-ray detector 200 will be described in detail.

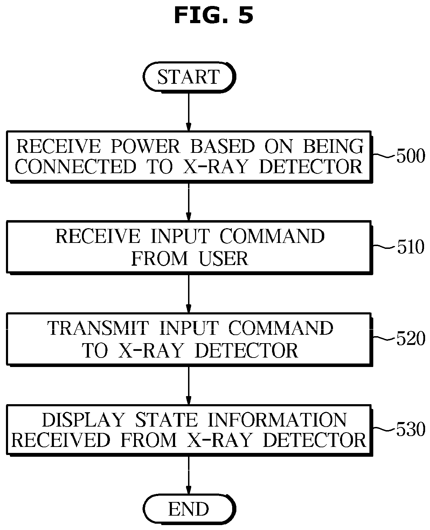

FIG. 5 is a flow chart illustrating an operation of the portable expansion module according to an embodiment of the disclosure.

Referring to FIG. 5, the portable expansion module 100 may receive power based on being connected to the X-ray detector 200 (S500).

Particularly, the portable expansion module 100 may receive power from the X-ray detector 200 via the connecting portion 130. The X-ray detector 200 may supply power to the portable expansion module 100 via the battery 210 provided in the X-ray detector 200.

The portable expansion module 100 may receive an input command from a user (S510).

The portable expansion module 100 may receive an input command from a user via the input component 120. The input command may include various commands such as an input command for the AED function, as well as a command for changing the Soft AP mode.

The portable expansion module 100 may transmit the received input command to the X-ray detector 200 (S520).

The portable expansion module 100 may convert the received input command into an electrical signal, and then transfer the converted electrical signal to the X-ray detector 200 via the connecting portion 130. The X-ray detector 200 may perform an operation based on the received input command.

The portable expansion module 100 may display state information received from the X-ray detector 200 (S530).

The state information may include the current state of the X-ray detector 200, state information based on the operation result after the operation based on an input command, or the like. The state information may be displayed on the display 110 of the portable expansion module 100, and the display 110 may display the state information including at least one of an AED standby state, the number of possible AED images, a patient ID, an imaging number, or the like.

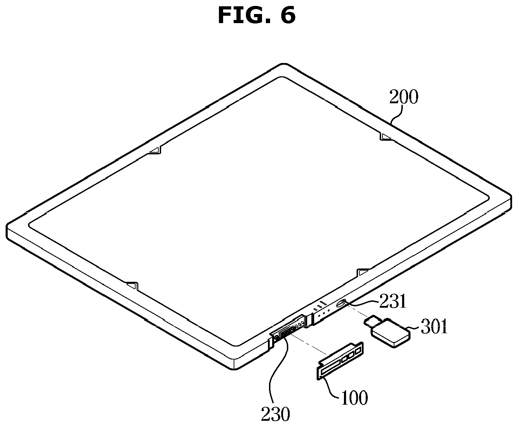

FIG. 6 is a view illustrating an embodiment in which the portable expansion module operates as a storage medium.

Referring to FIG. 6, the X-ray detector 200 may further include a second connector 231 to which various storage media such as a Universal Serial Bus (USB) drive 301 is inserted, in addition to the connector 230 connected to the portable expansion module 100 or the main cable 3. The user may store data generated by the X-ray detector 200 via the USB drive 301.

However, although not shown in FIG. 2A, the portable expansion module 100 may function as the storage medium such as the USB drive 301. Particularly, when the portable expansion module 100 is coupled to the connector 230, the X-ray detector 200 may transmit various data such as an X-ray image, in which the signal processing is performed, to the portable expansion module 100. In this case, the portable expansion module 100 may include a non-volatile memory device such as a ROM, a PROM, an EPROM, an EEPROM, and a flash memory.

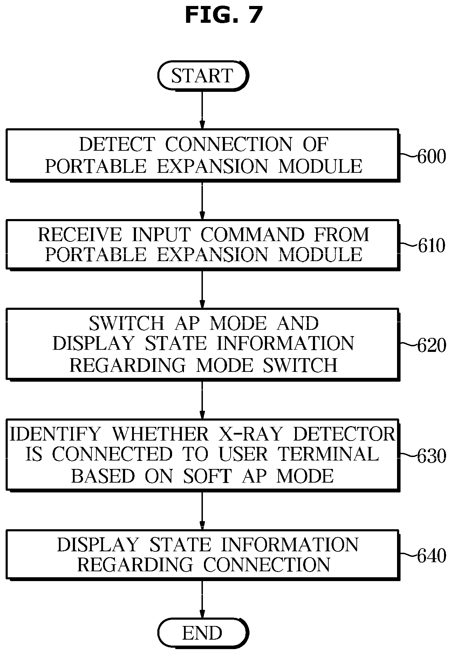

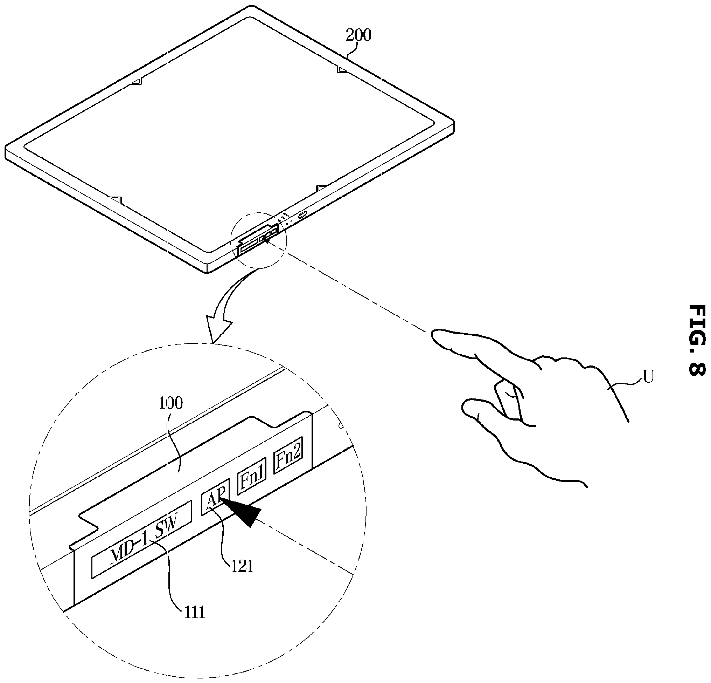

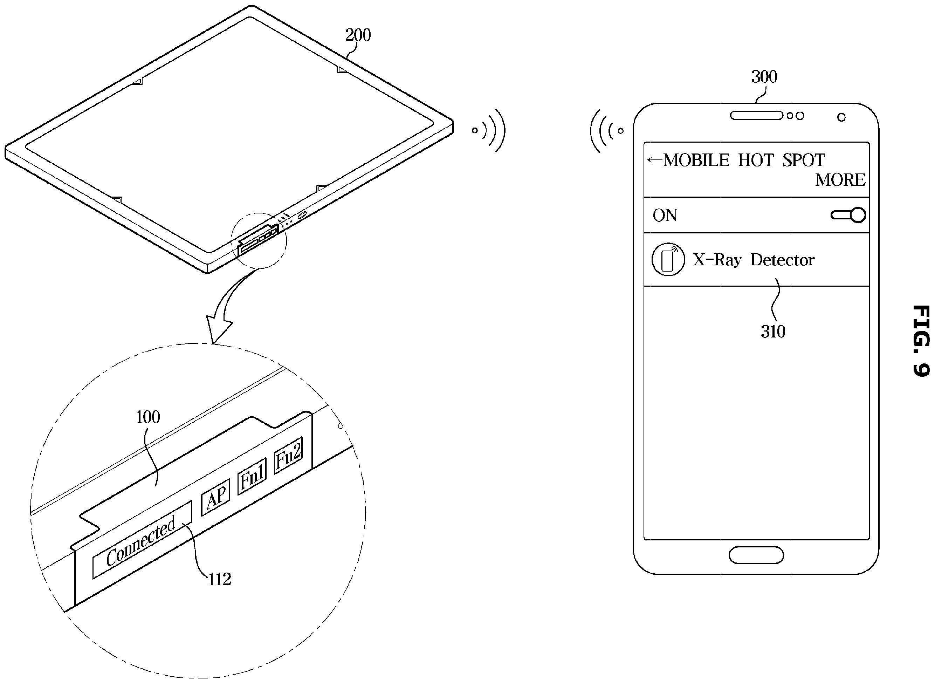

FIG. 7 is a flow chart illustrating a method for changing a communication mode of the X-ray detector, and FIGS. 8 and 9 are views illustrating an example of FIG. 7. In order to reduce redundant description, FIGS. 7, 8, and 9 will be described together.

Referring to FIG. 7, the X-ray detector 200 may detect the connection of the portable expansion module 100 (S600).

For example, based on the portable expansion module 100 being connected to the connector 230, the connector 230 may generate an electrical signal and transmit the generated electrical signal to the controller 250. The controller 250 may identify the connection of the portable expansion module 100 based on the detection result of the connector 230.

The X-ray detector 200 may receive an input command from the portable expansion module 100 (S610). For example, the portable expansion module 100 may receive an input command from a user, and may transmit the input command to the X-ray detector 200.

Referring to FIG. 8, a user U may select an AP button 121, which may be an input button for changing an AP mode, of the input component 120 of the portable expansion module 100 connected to the X-ray detector 200. The portable expansion module 100 may convert an electrical signal from an input command received from the AP button 121, and transmit the electrical signal to the controller 250 of the X-ray detector 200.

The X-ray detector 200 may switch the AP mode based on the input command, and may display state information regarding the mode switch via the portable expansion module 100 (S620).

As described above in FIG. 4, the X-ray detector 200 may switch the communication mode from the AP mode to the Soft AP mode based on the input command. In addition, after changing the communication mode to the Soft AP mode, the X-ray detector 200 may display state information regarding the mode switch on the display 110 of the portable expansion module 100 as shown in FIG. 8. For example, the X-ray detector 200 may display "MD-1 SW" corresponding to state information 111 that indicates that Mode-1, which means the Soft AP mode, is switched.

The X-ray detector 200 may identify whether the X-ray detector 200 is connected to the user terminal 300 based on the soft AP mode (S630).

As described above in FIG. 4, in the soft AP mode, the X-ray detector 200 may directly communicate with the user terminal 300. Therefore, in order for the X-ray detector 200 and the user terminal 300 to communicate with each other, the X-ray detector 200 and the user terminal 300 may confirm the interconnection.

Referring to FIG. 9, the X-ray detector 200 may transmit a search signal to the user terminal 300 via the communication circuitry 220, and the user terminal 300 may receive the search signal. The user terminal 300 may display a name 310 that refers to the X-ray detector 200 included in the search signal such as, for example, "X-Ray Detector." Based on the user inputting a command to select the name 310, the user terminal 300 may transmit a signal in response to the search signal to the X-ray detector 200. Accordingly, the X-ray detector 200 may confirm the interconnection with the user terminal 300.

Based on the connection being confirmed, the X-ray detector 200 may display state information indicating that the connection with the user terminal 300 is completed via the display 110 of the portable expansion module 100 (S640).

Referring to FIG. 9, the X-ray detector 200 may display "Connected" as state information 112 indicating that the interconnection is completed, via the display 110.

The embodiment shown in FIGS. 8 and 9 is an example of state information that is displayed regarding the switch of the communication mode, and thus the embodiment may further include various modifications.

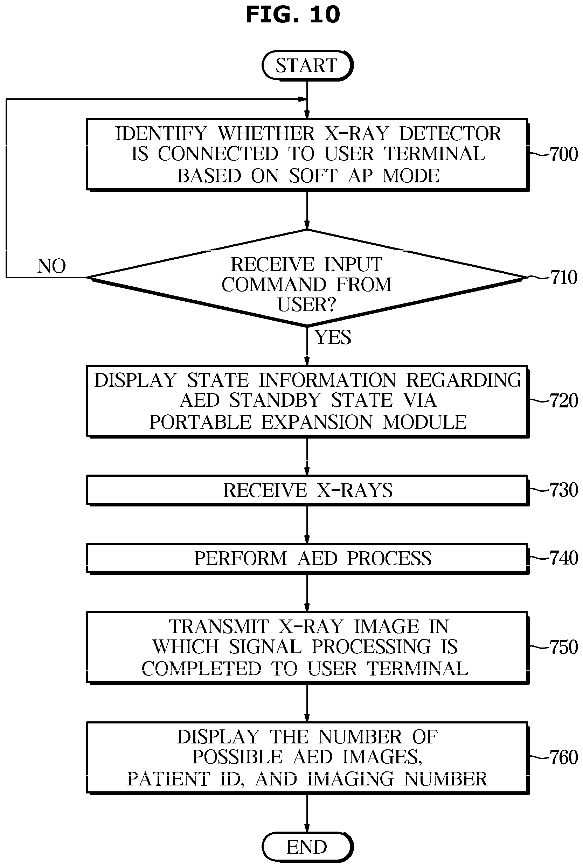

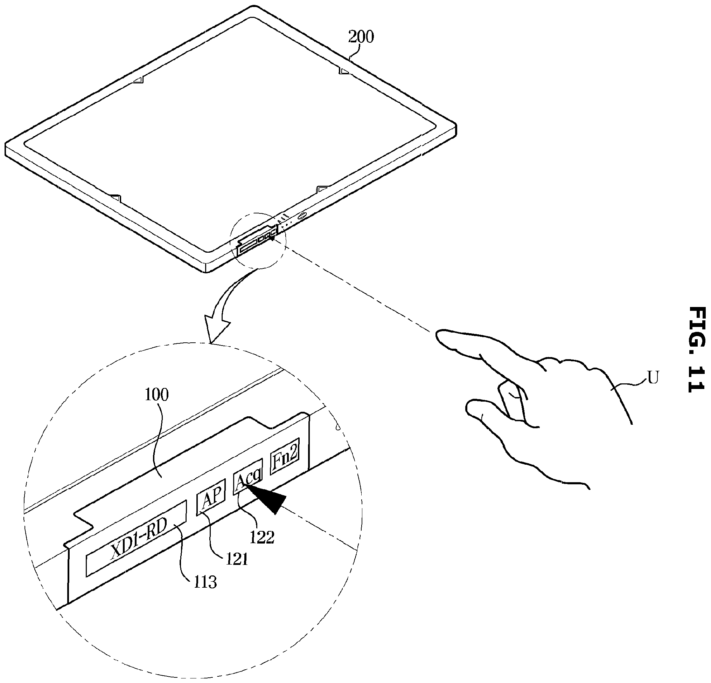

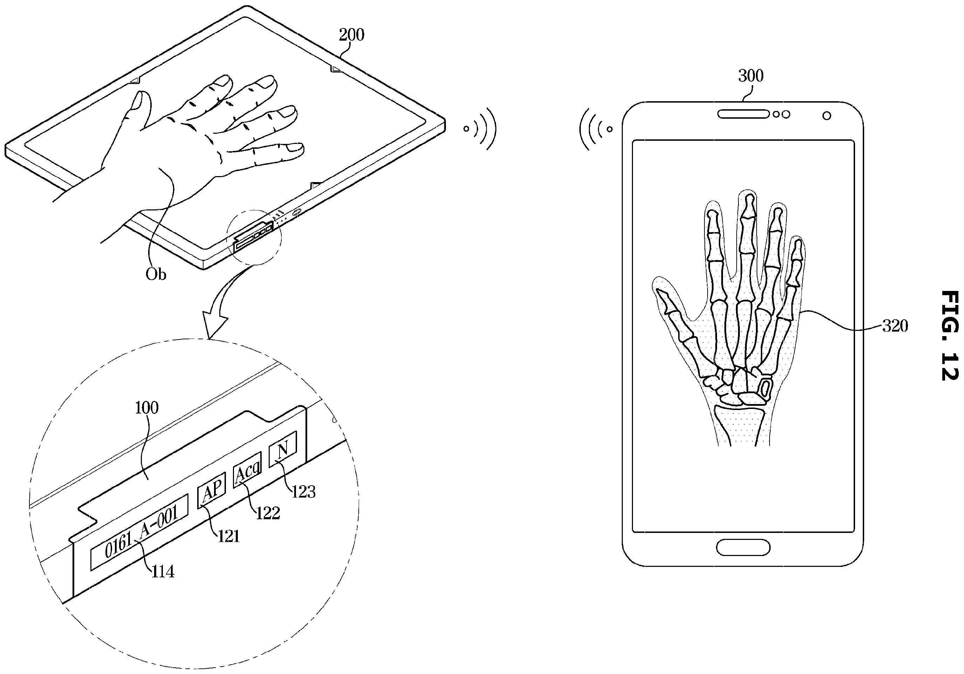

FIG. 10 is a flowchart illustrating an X-ray imaging method of the X-ray detector, and FIGS. 11 and 12 are views illustrating an example of FIG. 10. In order to reduce redundant description, FIGS. 10, 11, and 12 will be described together.

Referring to FIG. 10, the X-ray detector 200 may identify whether the X-ray detector 200 is connected to the user terminal 300 based on the Soft AP mode (S700).

After the connection is completed, the X-ray detector 200 may determine whether an input command is received from the user (S710).

Based on the portable expansion module 100 not receiving an input command, the X-ray detector 200 may continue to operate in a state of identifying whether the portable expansion module 100 is connected to the user terminal 300.

Alternatively, the X-ray detector 200 may receive an input command relating to the AED function. Referring to FIG. 11, among the input components 120 of the portable expansion module 100 connected to the X-ray detector 200, the user U may click an "Acq" button 122 corresponding to an input command for executing an AED function.

Based on the input command being received, the X-ray detector 200 may display state information regarding the AED standby state via the portable expansion module 100 (S720).

The X-ray detector 200 may execute the AED function. As described above, the AED function may refer to a state in which the X-ray detector 200 continuously monitors the radiated X-rays. Therefore, after the AED function is executed, the X-ray detector 200 may transmit state information indicating that the X-ray detector 200 is ready to receive X-rays via the detector 240. Referring to FIG. 11, the X-ray detector 200 according to an embodiment may display "XD1-RD" indicating state information 113 related to the AED standby state, on the display 110 of the portable expansion module 100. "XD1" may represent subsystem identification (SSID), and "RD" may stand for "Ready."

Based on the AED function being executed, the X-ray detector 200 may receive the X-rays (S730) and may perform an AED process (S740).

Particularly, the X-ray detector 200 may operate the detector 240 based on X-rays having a size that is greater than or equal to a predetermined size being irradiated. Referring to FIG. 12, the user U may place an object Ob for acquiring the X-ray image on the X-ray detector 200. Thereafter, the user U radiates the X-rays towards the object Ob using the X-ray source 10. Because the X-ray detector 200 is in the AED standby state, the X-ray detector 200 may operate the detector 240 based on detecting the X-rays. The detector 240 may finally generate X-ray data. The X-ray data may be converted into an X-ray image using a signal processing technique executed by the controller 250.

The X-ray detector 200 may transmit the X-ray image in which the signal processing is completed to the user terminal 300 (S750).

Particularly, the communication circuitry 220 may transmit the X-ray image to the user terminal 300 based on the Soft AP mode. The user terminal 300 may display the received X-ray image 320 as shown in FIG. 12.

After transmitting the X-ray image to the user terminal 300, the X-ray detector 200 may display state information such as the number of possible AED images, a patient ID, and an imaging number (S760).

Referring to FIG. 12, the X-ray detector 200 may assign a patient ID and an imaging number to the X-ray image to distinguish the generated X-ray image. In addition, the X-ray detector 200 may count the number of possible AED images.

For example, the X-ray detector 200 may display state information 114 such as "0161" indicating the number of possible images, "A" indicating the patient ID, and "001" corresponding to the imaging number, on the display 110 of the portable expansion module 100. Accordingly, the user U may identify whether the X-ray imaging processed by the current X-ray detector 200 is successful, and whether the X-ray detector 200 is available to perform the X-ray imaging later, and may distinguish an X-ray image displayed on the user terminal 300 based on the provided state information.

An input button (N) 123 of the input component 120 illustrated in FIG. 12 may represent an input command for collectively transmitting a plurality of X-ray images, which is acquired as a user continuously performs X-ray imaging on an object Ob several times, to the user terminal 300. That is, the user may transmit the plurality of generated X-ray images to the user terminal 300 using the "N" button 123, and may change the patient ID of the X-ray image displayed in the AED process to "B," and the display 110 may display "B".

The embodiment illustrated in FIGS. 11 and 12 is an example of displaying the state information based on the switching of the communication mode, and thus other embodiments may further include various modifications.

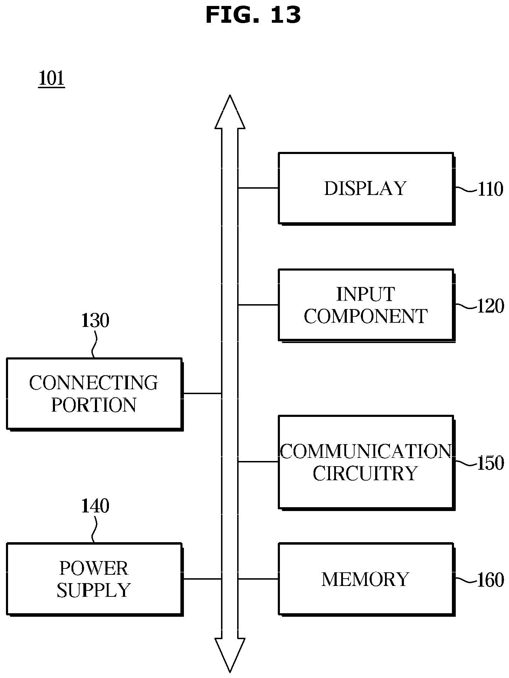

FIG. 13 is a control block diagram of a portable expansion module according to an embodiment of the disclosure.

Referring to FIG. 13, a portable expansion module 101 according to an embodiment may further include various configurations in addition to the display 110, the input component 120, and the connecting portion 130 described above with reference to FIG. 2A.

Particularly, the portable expansion module 101 may further include a power supply 140 configured to supply power, a communication circuitry 150 configured to communicate with a workstation 400, and a memory 160 configured to correspond to a storage medium.

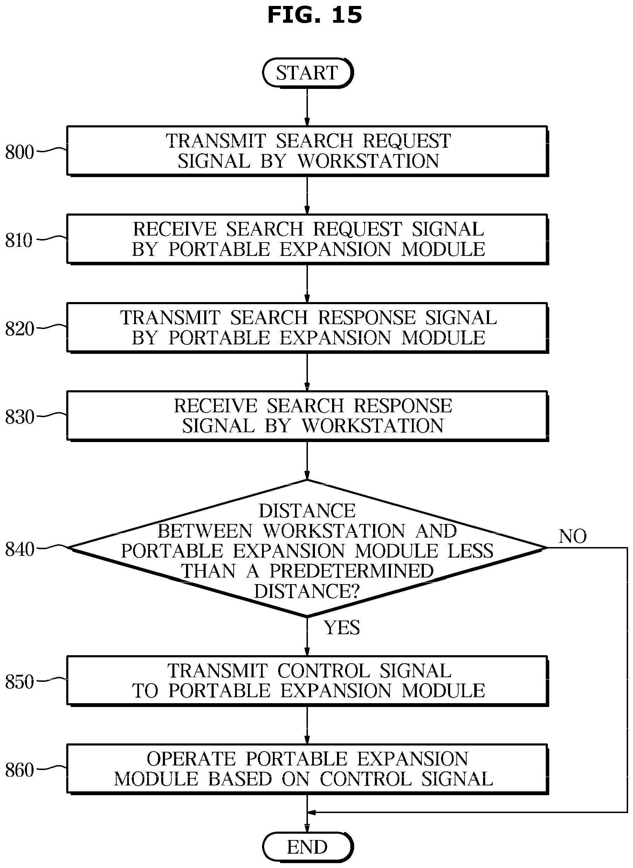

The power supply 140 may supply power to each component so that one component of the portable expansion module 101 according to an embodiment may operate without being connected to the X-ray detector 200. For example, the power supply 140 may supply power to the communication circuitry 150 to allow the workstation 400 to search for the portable expansion module 101. A detailed description thereof will be described in more detail elsewhere herein with reference to FIGS. 15 and 16.

The communication circuitry 150 may communicate with the workstation 400 within a limited distance, and may be a communication module such as a short-range communication module.

As described above with reference to FIG. 6, the memory 160 may allow the portable expansion module 101 to operate as a storage medium.

In addition, the portable expansion module 101 may further include various components configured to provide additional functionality, and the above-described components may be omitted.

FIG. 14 is an external view illustrating an X-ray imaging apparatus including the portable expansion module according to an embodiment of the disclosure.

The X-ray imaging apparatus 1 as shown in FIG. 14 may be a ceiling-type X-ray imaging apparatus in which a tube head unit is connected to the ceiling. However, the X-ray imaging apparatus 1 is not limited thereto, and may further include various embodiments such as a mobile X-ray imaging apparatus.

Referring to FIG. 14, a guide rail 30 may be installed on the ceiling of an examination room in which the X-ray imaging apparatus 1 is installed. A tube head unit 11 may be connected to a moving carriage 40 that moves along the guide rail 30, thereby permitting the tube head unit 11 to move to a position corresponding to an object to be imaged.

The guide rail 30 may include a first guide rail 31 and a second guide rail 32 installed to form a predetermined angle with respect to each other. For example, the first guide rail 31 and the second guide rail 32 may be installed to be perpendicular to each other.

The first guide rail 31 may be installed on the ceiling of the examination room, and the second guide rail 32 may be slidably mounted to the lower side of the first guide rail 31. A roller configured to move along the first guide rail 31 may be installed on the first guide rail 31. The second guide rail 32 may be connected to the roller, and may move along the first guide rail 31.

A first direction D1 may be defined as a direction in which the first guide rail 31 extends, and a second direction D2 may be defined as a direction in which the second guide rail 32 extends. Therefore, the first direction D1 and the second direction D2 may be perpendicular to each other, and may be parallel to the ceiling of the examination room.

The moving carriage 40 may be arranged below the second guide rail 32 and may be movable along the second guide rail 32. A roller configured to move along the second guide rail 32 may be installed in the moving carriage 40. Therefore, the moving carriage 40 may be movable in the first direction D1 together with the second guide rail 32, and may be movable in the second direction D2 along the second guide rail 32.

A post frame 50 may be connected to a lower portion of the moving carriage 40. The post frame 50 may include a plurality of posts 51, 52, 53, 54, and 55.

Because the plurality of posts 51, 52, 53, 54, and 55 may be foldably connected to each other, the post frame 50 may be shortened in the upper direction of the examination room or extended in the lower direction while being fixed to the moving carriage 40.

Because the tube head unit 11 is connected to the lower end of the post frame 50, a height from the ground to the tube head unit 11 may be controlled by the expansion and contraction of the post frame 50.

A third direction D3 may be defined as a direction in which the length of the post frame 50 is extended or shortened. Therefore, the third direction D3 may be perpendicular to the first direction D1 and the second direction D2.

The tube head unit 11 may be an X-ray source 10 configured to irradiate an X-ray towards an object. The tube head unit 11 may be an assembly including an x-ray tube configured to generate X-rays, and a collimator configured to adjust an irradiation range of the generated X-rays

The tube head unit 11 may be connected to the moving carriage 40 via a connecting pipe 75. Various cables and wires configured to connect the tube head unit 11 to other devices may be built in the connecting pipe 75, and a high voltage generated by a high voltage generator may also supplied to the tube head unit 11 via the connecting pipe 75.

A rotary joint 60 may be arranged between the tube head unit 11 and the post frame 50. The rotary joint 60 may connect the tube head unit 11 to the post frame 50, and may support the load applied to the tube head unit 11.

The rotary joint 60 may include a first rotary joint 61 connected to a lower post 51 of the post frame 50, and a second rotary joint 62 connected to the tube head unit 11.

The first rotary joint 61 may be configured to be rotatable about a central axis of the post frame 50 extending in the vertical direction of the examination room. Therefore, the first rotary joint 61 may rotate on a plane perpendicular to the third direction D3. In this case, a rotation direction of the first rotary joint 61 may correspond to a fourth direction D4 that is a rotation direction of an axis parallel to the third direction D3.

The second rotary joint 62 may be configured to be rotatable on a plane perpendicular to the ceiling of the examination room. Accordingly, the second rotary joint 62 may be rotatable in a rotational direction of an axis parallel to the first direction D1 or the second direction D2. At this time, the rotation direction of the second rotary joint 62 may correspond to a fifth direction D5, and may be a rotation direction of an axis extending in the first direction or the second direction.

As the tube head unit 11 is connected to the rotary joint 60, the tube head unit 11 may perform the rotational movement in the fourth direction D4 and the fifth direction D5. When the second rotary joint 62 rotates in the fifth direction D5, a tilt angle of the tube head unit 11 may be adjusted.

In addition, the tube head unit 11 may be connected to the post frame 50 via the rotary joint 60 and then linearly move in the first direction D1, the second direction D2, and the third direction D3.

A tube motor 90 may be provided to move the tube head unit 11 in the first, second, third, fourth and fifth directions D1, D2, D3, D4, and D5, and the tube motor 90 may include an encoder configured to measure a rotation speed thereof.

The tube motor 90 may include a plurality of motors 91, 92, and 93 corresponding to the first, second, and third directions D1, D2, and D3, and each motor may be arranged at various positions in consideration of design convenience.

For example, a first motor 91 configured to move the second guide rail 32 in the first direction D1 may be arranged around the first guide rail 31, a second motor 92 configured to move the moving carriage 40 in the second direction may be arranged around the second guide rail 32, and a third motor 93 configured to increase or decrease the length of the post frame 50 in the third direction D3 may be arranged in the moving carriage 40.

Further, a motor configured to rotate the tube head unit 11 in the fourth direction D4 may be arranged around the first rotary joint 61, and a motor configured to rotate the tube head unit 11 in the fifth direction D5 may be arranged around the second rotary joint 62.

Each motor may be connected to a power transmission means to linearly or rotationally move the tube head unit 11 in the first direction D1 to the fifth direction D5. The power transmission means may include belts and pulleys, chains and sprockets, shafts, etc.

A control panel 80 configured to provide information to the user and receive a control command from the user may be provided on one side of the tube head unit 11.

An imaging table 22 and an imaging stand 21 on which the X-ray detector 200 is mounted may be provided at a position adjacent to the moving range of the tube head unit 11.

A detector mounting portion 22a may be formed in a lower portion of the imaging table 22, and the detector mounting portion 22a may be movable in a longitudinal direction (direction D8) of the imaging table 22. When the X-ray detector 200 is inserted into the detector mounting portion 22a and the object is positioned on the upper portion of the imaging table 22, the tube head unit 11 and the detector mounting portion 22a may be moved to a position corresponding to an imaging part of the object and perform the X-ray imaging.

A detector mounting portion 21a may also be formed in the imaging stand 21, and the detector mounting portion 21a may also movable in the longitudinal direction (direction D6) of the imaging stand 21. The longitudinal direction of the imaging stand 21 may be perpendicular to the longitudinal direction of the imaging table 22. When the X-ray detector 200 is inserted into the detector mounting portion 21a and the object is positioned in front of the detector mounting portion 21a, the tube head unit 11 and the detector mounting portion 21a may be moved to a position corresponding to the imaging portion of the object and perform the X-ray imaging.

Although not shown, the X-ray imaging apparatus 1 may include a motor configured to move the detector mounting portion 22a of the imaging table 22 in the direction D8, and a motor configured to move the detector mounting portion 21a of the imaging stand 21 in the direction D6.

The X-ray imaging apparatus 1 may include a host device configured to control an overall operation of the X-ray imaging apparatus 1. As an example, the host device may include a workstation 400 as shown in FIG. 14. The workstation 400 may be located in a space separated by a partition B from a space where the tube head unit 11 is placed.

The workstation 400 may include a workstation display 440 configured to display X-ray images, a screen for guiding the input of control commands, and various setting information related to the X-ray imaging apparatus 1, and a workstation input component 450 configured to receive various control commands related to X-ray imaging from a user.

According to an embodiment, a user may be separated from the workstation 400 and prepare for the X-ray imaging with the object by using the X-ray detector 200 in a location in which the tube head unit 11 is arranged. However, because the user may not know the location of the portable expansion module 101, the user may search for the portable expansion module 101. In this case, the user may transmit a search request signal to the portable expansion module 101 via the workstation 400. In this way, the user may confirm that the portable expansion module 101 is placed on the imaging stand 21.

FIG. 15 is a flow chart illustrating a function provided by the portable expansion module according to an embodiment.

Referring to FIG. 15, the workstation 400 may transmit a search request signal (S800).

Because the portable expansion module 101 according to an embodiment includes the power supply 140 and the communication circuitry 150, the portable expansion module 101 may operate without being connected to the X-ray detector 200.

Based on receiving the search request signal (S810), the portable expansion module 101 may transmit a search response signal corresponding to the search request signal (S820). The workstation 400 may receive the search response signal transmitted by the portable expansion module 101 (S830).

The workstation 400 may identify, based on the search response signal, whether a distance between the portable expansion module 101 and the workstation 400 is less than a predetermined distance (S840).

For example, the workstation 400 may identify a distance of the portable expansion module 101 based on the strength of the search response signal.

Based on the portable expansion module 101 being within a predetermined distance, the workstation 400 may transmit a control signal to the portable expansion module 101 (S850).

The portable expansion module 101 may operate based on the control signal (860).

For example, the portable expansion module 101 may operate the display 110 to allow a user to identify the location of the portable expansion module 101. By emitting light from the display 110, the portable expansion module 101 may permit a user to readily locate the portable expansion module 101. As another example, the portable expansion module 101 may output a sound to permit the user to readily locate the position of the portable expansion module 101 based on the sound output by the portable expansion module 101.

Accordingly, the portable expansion modules 100 and 101 may provide additional functionality to the X-ray detector 200, and may increase the usability and mobility of the wireless X-ray detector 200 because the portable expansion modules 100 and 101 allow the X-ray detector 200 to directly communicate with a user terminal 300 other than the workstation 400, and allow the user terminal 300 to provide a service provided by the workstation 400.

As is apparent from the above description, the portable expansion module 100 or 101 may provide additional functionality to the X-ray detector.

It is possible to increase the usability and mobility of the wireless X-ray detector 200 because the X-ray detector 200 may directly communicate with a user terminal 300 other than the workstation 400, and the user terminal 300 may provide a service that has been provided by the workstation 400.

Although embodiments of the disclosure have been shown and described, it should be appreciated by those skilled in the art that changes may be made to these embodiments without departing from the principles of the disclosure, the scope of which is defined in the claims and their equivalents.

## Claims

1. A portable expansion module comprising:
a connecting portion configured to connect to a connector of an X-ray detector; and
a display configured to:
operate using power supplied from the X-ray detector based on the connecting portion being connected to the connector;
receive data from the X-ray detector based on the connecting portion being connected to the connector; and
display state information of the X-ray detector based on the data received from the X-ray detector.

2. The portable expansion module of claim 1, further comprising:
an input component configured to receive an input command from a user,
wherein based on the connecting portion being connected to the connector, the input component is configured to transmit the input command identifying a change in a software enabled access point (Soft AP) mode to the X-ray detector.

3. The portable expansion module of claim 1, further comprising:
an input component configured to receive an input command from a user,
wherein the input component is configured to transmit the input command identifying an Automatic Exposure Detection (AED) function to the X-ray detector.

4. The portable expansion module of claim 1, wherein the display is further configured to:
display state information including at least one of a switching of a Soft AP mode of the X-ray detector, an AED standby state, a number of possible AED images, a patient identifier (ID), and an imaging number, based on the data received from the X-ray detector.

5. The portable expansion module of claim 1, further comprising:
a memory configured to store the data received from the X-ray detector.

6. The portable expansion module of claim 1, further comprising:
a communication circuitry configured to:
receive a search request signal from a workstation; and
transmit a search response signal in response to the search request signal to the workstation.

7. An X-ray detector comprising:
a battery;
a connector configured to connect to at least one of a cable or a portable expansion module;
a detector configured to detect X-rays that are irradiated from an X-ray source; and
a controller configured to:
detect that the X-ray detector is connected to the portable expansion module;
supply power to the portable expansion module from the battery based on detecting that the X-ray detector is connected to the portable expansion module; and
transmit data associated with state information of the X-ray detector to the portable expansion module to permit the portable expansion module to display the state information.

8. The X-ray detector of claim 7, wherein the controller is further configured to:
switch from an access point (AP) mode to a software enabled access point (Soft AP) mode based on detecting that the X-ray detector is connected to the portable expansion module.

9. The X-ray detector of claim 7, wherein the controller is further configured to:
switch from the AP mode to the Soft AP mode based on an input command transmitted via the portable expansion module.

10. The X-ray detector of claim 7, wherein the controller is further configured to:
control the detector to perform an Automatic Exposure Detection (AED) function based on an input command transmitted via the portable expansion module; and
display state information regarding an AED standby state via the portable expansion module.

11. The X-ray detector of claim 10, wherein the controller is further configured to:
convert X-rays detected by the detector into an X-ray image, based on the AED function; and
display state information identifying a completion of the X-ray imaging, via the portable expansion module.

12. The X-ray detector of claim 11, further comprising:
a communication circuitry configured to communicate with a user terminal that is external to the X-ray detector,
wherein the controller is further configured to identify the user terminal that is connected to the X-ray detector via the communication circuitry, based on the Soft AP mode.

13. The X-ray detector of claim 12, wherein the controller is further configured to:
transmit the X-ray image to the user terminal via the communication circuitry.

14. The X-ray detector of claim 11, further comprising:
a storage configured to store the X-ray image,
wherein the controller is further configured to assign an imaging number to the X-ray image stored in the storage.

15. The X-ray detector of claim 14, wherein the controller is further configured to:
display state information including at least one of a number of possible AED images, the imaging number, and a patient identifier (ID) associated with the stored X-ray image, via the portable expansion module.
